# EUROPEAN PATENT SPECIFICATION

(11) **EP 3 210 014 B1**
(45) Date of publication and mention of the grant of the patent: **03.08.2022**
(21) Application number: 15797864.4
(22) Date of filing: 20.10.2015
(51) Int. Cl.: G01N 33/483

(54) **A DEVICE COMPRISING A MULTI ELECTRODE ARRAY (MEA)**
MEHRFACHELEKTRODENANORDNUNG (MEA)
RÉSEAU D'ÉLECTRODES MULTIPLES (MEA)

(30) Priority: 20.10.2014 EP 14189581
(43) Date of publication of application: 30.08.2017
(73) Proprietor: Haute Ecole d'Ingénierie et de Gestion du Canton de Vaud (HEIG-VD), 1401 Yverdon-les-bains (CH)
(72) Inventor: BOVEY, Dominique, CH-1613 Maracon (CH); COEUDEVEZ, Pascal, CH-2300 La Chaux-de-Fonds (CH); GRAVIER, Laurent, CH-1024 Ecublens (CH)
(74) Representative: Schneiter, Sorin
(86) International application number: PCT/EP2015/074283
(87) International publication number: WO 2016/062729

(56) References cited:
- EP-A1- 2 581 742
- EP-A1- 2 639 576
- EP-A1- 2 677 307
- WO-A1-2014/085727
- US-A1- 2004 106 101
- US-A1- 2010 120 626
- LINDBERG M ET AL: "A comprehensive study of ion track enabled high aspect ratio microstructures in flexible circuit boards", MICROSYSTEM TECHNOLOGIES, BERLIN, DE, vol. 10, 2004, pages 608-621, XP003020386, ISSN: 0946-7076, DOI: 10.1007/S00542-003-0339-2

## Description

### Technical Field

The present invention relates to a device comprising a multielectrode array or microelectrode array (MEA), to methods of measuring impedance and/or potential differences between array electrodes of a MEA, to the use of ion track etched membranes in the preparation of a MEA.

### Prior Art and the Problem Underlying the Invention

Multi electrode array or microelectrode array (MEA) biochips are used in biology and medical research for studying and monitoring biological preparations containing living cells. The biochip contains generally a well for the sample and a plurality of electrodes, generally dot electrodes provided in the form of an array on the substantially planar bottom surface of the well. Electrodes can also be on a concave wall bottom surface or on the inner surface of the wall of the well. In general, MEA biochips allow the direct measurement of electrical activity of the biological preparation. Non-implantable, *in vitro* MEA applications, can be used, for example, for measuring impedance between electrodes of the MEA. This allows, for example, monitoring viability or proliferation of cells of a preparation or sample, because, generally, only living cells adhere to the inner surfaces of the well and thus are at the origin of changes with respect to impedance. On the other hand, MEA biochips are also used to monitor action potentials of excitable cells, such as neurons, muscle cells and endocrine cells. In particular, MEA biochips can be used for measuring a difference of potential between two electrodes of the MEA, allowing the detection of an action potential of a cell that is in vicinity of an electrode. In this context, MEAs can be used to measure extracellular field potential, derived from intracellular potential. By applying an external potential, MEAs can further be used to induce action potential in excitable cells. MEA biochips are frequently used as biosensors, for example in drug screening.

The performance of a MEA biochip depends on several parameters, such as the size of the dot electrodes, the distance between the electrodes and the density of electrodes on a given surface of the well containing the MEA. Today's challenges reside in the optimization of the geometry of MEA electrodes, reducing the internal impedance of the circuit and electrodes, and reducing noise.

It is an objective of the present invention to reduce the resistance of the circuit and or electrodes of a MEA biochip. It is a further objective to improve the analog signal processing, such as the system's gain, bandwidth, and behavior outside of cutoff frequencies data. It is also an objective to increase the sampling rate, the digital signal processing and the sampling properties in general. Further objectives of the invention are to decrease the amount of data produced during measurements through signal compression and feature extraction using digital signal processing.

When operating MEA biochips, there are important challenges with respect to the production of data and the management thereof. Hardware components and computers are generally directly connected to the MEA biochip management system, so as to allow transfer of all generated data to the computer for data analysis. As a consequence, MEA biochips are generally cumbersome and it is not easy to handle large numbers of samples simultaneously. A particular objective of the invention is to provide a multi-MEA cell culture analysis apparatus that can be placed directly in an incubator.

In addition, current implementations of MEAs are relatively expensive. It would be advantageous to reduce the costs of the MEA biochips and to reduce waste, given that a MEA biochip is generally used only once.

It is an objective of the invention to provide a cost-effective cell culture apparatus, allowing automated monitoring of the cell health, crucial to the development of large scale toxicological testing on cell cultures. The latter should allow decreasing the number of toxicity tests performed *in vivo* on living animals.

EP 2639576A1 discloses a real-time electronic cell sensing system with multiple electrode arrays, wherein on electrode array comprises two electrodes, wherein different electrode arrays share a common connection pad.

US 2010/0120626 discloses multiwell MEAs wherein each well comprises up to 768 electrodes and the substrate of the MEA may be transparent.

EP 2677307A1 discloses an integrated circuit (IC) with wells for monitoring DNA sequencing, the IC comprising sensing electrodes suitable to determine the pH of the medium in the wells, wherein each well is suitable to receive a bead functionalized with a DNA sequencing primer having a particular sequence.

The present invention addresses the problems depicted above.

### Summary of the Invention

Remarkably, the present invention provides novel MEAs and devices comprising MEAs that address the problems described above.

The present invention provides a device comprising one or more wells adapted to receive a sample with cells, wherein each well comprises a multielectrode array (MEA), wherein said MEA comprises a membrane on which a plurality of array electrodes are arranged to form the array of said MEA, wherein said membrane comprises an upper surface on which said array electrodes emerge, and a lower surface where said membrane is in contact with electronic circuit connecting the array electrodes to electronic equipment assisting in the operation of the MEA, wherein the membrane comprises groups of end-to-end nanoholes containing conductive nanolines, and wherein one group of conductive nanolines comprises more than 1000 nanolines and electrically connects one single array electrode of said MEA to said circuit on the lower surface of said membrane, and in that said electronic circuit is provided on an electronic circuit support board, wherein said membrane is attached on said board so as to electrically connect an array electrode to said circuit board via said conductive nanolines in a group of nanoholes.

In an embodiment, the device is a displaceable, portable and/or hand-held device.

In an embodiment, the device comprises a measuring subassembly for measuring electric characteristics of a sample in said well; a battery for providing electric energy to the electronic components of the device; one or more switches or selectors for selecting electrodes of said MEA in said well for the purpose of conducting measurements of electric characteristics of a sample in said well; a controller, configured to communicate with a computer via wireless data transmission and for activating said one or more switches, so as to determine the array electrodes of a MEA for conducting measurements; a wireless communication module enabling wireless communication between said controller and said computer; a temporary storage configured to compress data received from said measuring subassembly, before transmission of the data to said computer via said wireless communication.

In an embodiment, said MEA is produced by a method comprising the steps of: (1) providing a precursor membrane on a circuit board, the circuit board containing conducting lines; (2) producing nanopores on appropriate positions in the precursor membrane while the precursor membrane is attached to the circuit board, thereby obtaining a membrane containing nanopores; (3) producing nanolines inside said nanopores from the bottom of the nanopores to the upper surface of the membrane; and, (4) forming array electrodes of said MEA on the top surface of said MEA, thereby obtaining a MEA, wherein an individual array electrode of said MEA is formed by a group comprising more than 1'000 of nanolines.

In an embodiment, the device of the invention is suitable for conducting measurements, by measuring an electrical characteristic of a sample comprising living cells, the sample being provided in a well of the device of the present invention.

Further preferred embodiments of the invention are defined herein below and in the appended claims. Further features and advantages of the invention will become apparent to the skilled person from the description of the preferred embodiments below.

### Brief Description of the Drawings

**Figures 1** **A** and **B** schematically illustrate the ion-track etching method for producing membranes in accordance with an embodiment of the present invention.
**Figure 2** shows an electron microscope image of the surface of a nanoperforated membrane that can used for producing a MEA in accordance with an embodiment of the present invention. The image shown in Fig. 2 has been taken from the Internet page of Millipore AG.
**Figures 3** **A** to **3 D** schematically illustrate the formation of nanowires by electrochemical deposition in nanoperforated membranes illustrated in Fig. 2 and obtained as shown in Fig. 1.
**Figure 4** is a cross-section of a membrane containing electrodes, said membrane being part of the MEA the device according to of an embodiment of the present invention.
**Figures 5** **A** to **5 C** schematically illustrates a method for preparing a MEA for the device in accordance with an embodiment of the invention.
**Figures 6** **A** to **6 F** schematically illustrates a method for preparing a MEA in which each array electrodes comprise a bundle of nano stubbles.
**Figures 7** **A** and **B** are schematic representations of a system comprising portable devices for analyzing living cells or liquids in accordance with an embodiment of the present invention.
**Figure 8** is a more detailed schematic representation of a system for analyzing living cells or liquids, the system comprising the device in accordance with an embodiment of the present invention.
**Figure 9** is an extract of Figure 8, showing in more detail the MEA of the device of an embodiment of the present invention.

Hereinafter, preferred embodiments of the device of the invention will be described with reference to the drawings.

### Detailed Description of the Preferred Embodiments

In an embodiment, the device of the present invention is suitable for assisting in the operation of the MEAs. In the present invention, said membrane comprises an upper surface on which said array electrodes emerge. The membrane is preferably planar, comprising two opposed main surfaces, an upper or top surface and a lower or bottom surface. The membrane comprises groups of end-to-end nanoholes containing conductive nanolines for electrically connecting the electrode on the upper surface with an electronic circuit provided on the lower surface of the membrane. Said lines cross the membrane from the top to the bottom. In an embodiment, the MEA is a non-implantable MEA. In accordance with this embodiment, the MEA is preferably adapted for use with *in vitro* applications.

In accordance with another embodiment, the MEA is an implantable MEA. In accordance with this embodiment, the MEA is preferably adapted to *in vivo* applications. The MEA may be implantable to be in contact with neurons and/or be implanted to measure neural signals, for example electric signals produced by neurons. The invention also envisages a MEA that can be used for *in vitro* and *in vivo* applications.

For the purpose of this specification, an electrode of the MEA is referred to herein as a "MEA electrode" or "array electrode". These expressions differentiate the electrodes that are part of the MEA from other electrodes that may be present in the device of the invention, such as wall and/or reference electrodes. Preferably, the wall electrode and the reference electrode are not part of the MEA. Array electrodes may be dot electrodes or more generally "bottom" electrodes. In some embodiments, an individual array electrode may be (or all array electrodes may be, individually) in the form of a brush of nanowires, as will be described in more detail elsewhere in this specification.

To the best knowledge of the present inventors, membranes as described are not known to be used in MEAs. The production of membranes containing nanowires is generally disclosed, for example, in: H. Yousef et al. "Plated through-hole vias in a porous polyimide foil for flexible printed circuit boards", (2007) J. Micromech. Microeng. 18 (2008) 017001; and, M. Lindeberg et al "A comprehensive study of ion track enabled high aspect ratio microstructures in flexible circuit boards" Microsystem Technologies 10 (2004) 608-621; M. Lindeberg et al. "High aspect ratio 'multiple wire' microvias in flexible PCBs", (2009) Circuit World, 35, 4, pp. 18-21. The prior art is silent with respect to the use of membranes as disclosed in this specification as MEAs.

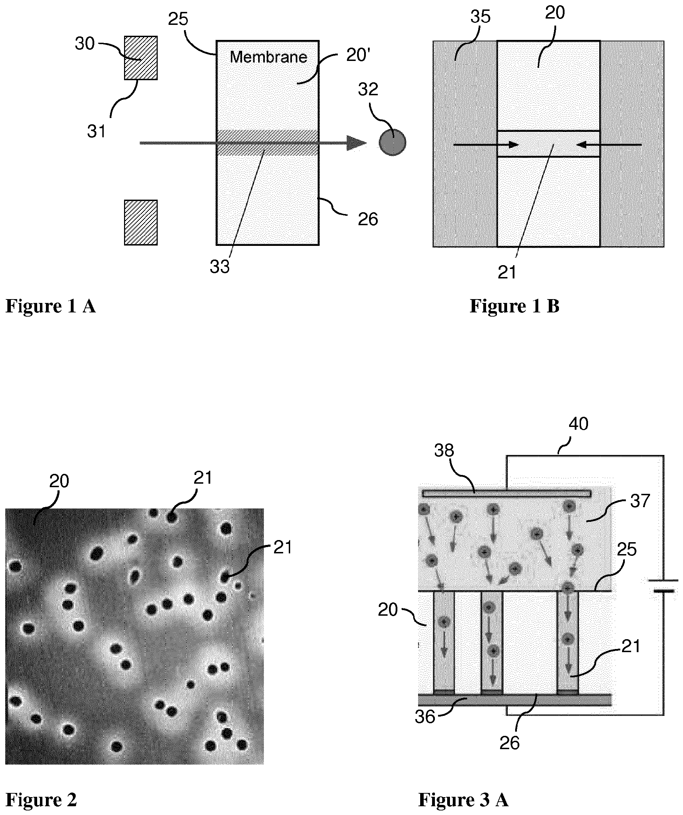

A suitable way for producing membranes that can be used for the purpose of the present invention is schematically disclosed in Figures 1 A and B.

In a preferred embodiment, the end-to-end holes in said membrane are made by ion track lithography.

In **Figure 1** **A,** one can see a planar mask 30 placed in front of a preferably planar precursor membrane 20'. In Fig. 1 A, only an extract of the mask 30 as well as of the membrane 20' is seen in cross section. Due to the planar configuration, the membrane has two opposing sides 25, 26. Further below, one side of said two opposing sides will be an upper side and the other side will be a lower side. However, it is not relevant which of the two sides 25, 26 is oriented towards the source of ions in Fig. 1.

The mask 30 comprises a plurality of openings, one of which is shown in Fig. 1 A with reference numeral 31. The mask 30 contains as many openings as desired or required for producing, for example, a MEA that may be used in accordance with the present invention. As will be understood, each opening 31 in the mask determines the position of a future array electrode in the membrane 20 discussed below in more detail. Accordingly, the distances and number of the openings 31 in the mask 30 are selected depending on the distance of the MEA electrodes of the MEA to be produced. Generally, for one opening 31 in the mask 30 a group or plurality of ions will pass, generating a group of ion tracks 33 in the membrane. The mask 30 has the purpose of defining the positions of the groups of ion tracks on the precursor membrane 20'. As will become apparent further below, each opening 31 will give rise to a group of nanopores and, eventually, to a group of nanowires that are associated with a particular array electrode.

The precursor membrane 20' is preferably made from a suitable material that is susceptible to ion track techniques. Preferably, the precursor membrane 20' and, consequently, the membrane 20, is made from an insulating material and/or comprises an insulating layer. As will become apparent to the skilled person, the membrane 20 containing nanowires 22 is preferably suitable to electrically separate the array electrodes of a MEA. If the membrane material is coated, the coating preferably extends on the inner surfaces of the nanopores 21. In a preferred embodiment, the precursor membrane 20', and therefore the membrane 20, comprises or consists essentially of a material selected from the group consisting of polycarbonate, polyimide, quartz, mica, glass, semiconductors and alumina (aluminum oxide).

In an embodiment, the membrane 20, 20' comprises or consists essentially of a polymer, for example selected from the group consisting of polycarbonate and polyimide.

The precursor membrane 20' covered by the mask 30 is preferably exposed to a source of ions creating an ion track 33 in the precursor membrane 20'. The ion track (or latent ion track) may be understood as damage anisotropy in the precursor membrane 20'. Since the ions 32 are preferably irradiated in a direction that is perpendicular to the surface 25 of the membrane, the ion tracks 33 extend in a perpendicular direction across the precursor membrane 20', as illustrated by the arrow in Fig. 1 A. Although this is not shown in Fig. 1 A, a plurality of essentially parallel ion tracks 33 is typically obtained for each opening 31 in the mask. The numbers of ion tracks can be adjusted by the ion source, exposure time, surface of the opening 31, for example.

**Figure 1** **B** illustrates the production of nanopores or nanoholes 21 in the precursor membrane 20' by wet etching. The precursor membrane 20' is exposed to a suitable etching liquid 35, which attacks chemically the material in the ion track 33, thereby producing parallel pores 21 in place of the ion track 33. **Figure 2** shows exemplary membrane 20 containing pores 21 following the wet etching process.

In an embodiment, the membrane 20 has a thickness in the range of 0.1 µm to 0.6 mm, preferably 1 µm to 300 µm, even more preferably 5 µm to 150 µm, and most preferably 10 µm to 100 µm. Preferably, the thickness is in the range of 20-70 µm. In a preferred embodiment, the thickness of the membrane is in the range of 60-140 µm, preferably 75-125 µm. In this regard, the membrane 20 is preferably thinner than typical circuit boards (generally a printed circuit board, PCB), which have a thickness of generally greater than 0.1 mm, generally even greater than 0.3 mm and in particular greater than about 0.5 mm, for example 0.5 up to 1mm. The thickness of the membrane essentially corresponds to the lent hog the nanowires within the membrane.

The term "nanoholes" or "nanopores" preferably refers to holes having a mean diameter below one micron. Preferably, all nanoholes have a diameter below one micron (< 1 µm). In an embodiment, the nanoholes have a diameter of 10 nm to 5 µm, preferably 15 nm to 1 µm, more preferably 20-700 nm, even more preferably 30- 300 nm, and most preferably 50-150 nm. Generally, the nanoholes have mean diameters in the range of 70-120 nm. Mean diameter may be determined, for example, from measuring diameters on electron microscope images such as the one shown in Fig. 2.

A conductive nanoline or nanowire generally fills the entire nanohole in which it is contained.

Therefore, the size (mean diameter) of the nanopores essentially corresponds to the thickness of the wires that will be contained in the pores, as will be described herein below. The nanolines thus have the thicknesses and preferred thicknesses indicated above.

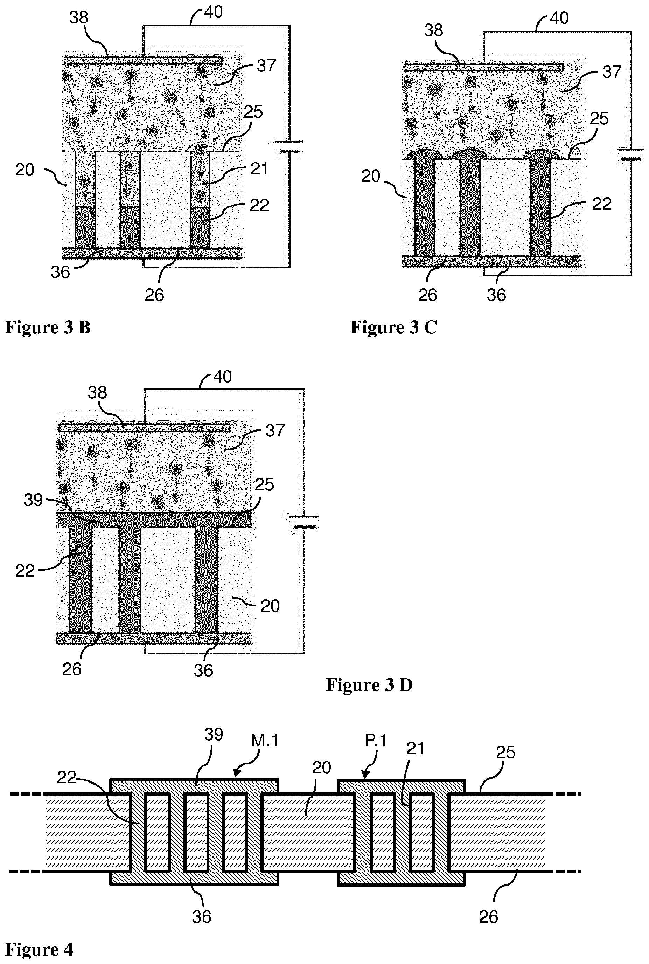

**Figures 3** **A** through **D** illustrate the production of nanowires 22 in the nanopores 21 and the formation of electrodes, such as M.1 and P.1 (Fig. 4) on the upper surface 25 of the membrane 20. In a first step, which is not illustrated in the figures, a conductive layer 36 is deposited on one side of the membrane. This may be done by physical vapor deposition (PVD) using a mask, such as the same mask 30 as shown in Fig. 1 A. Accordingly, the conductive layer 36, for example gold, is deposited on the membrane 20 at the positions where groups of nanopores had been created in previously described steps. The conductive layer 36 may be deposited on either side of the membrane, but is shown in the figures to be deposited on the lower surface 26.

The nanowires 22 are formed by metallization. Figs. 3 A through D illustrate the metallization by electrodeposition, such as galvanostatic or potentiostatic electrodeposition, for example. An electrochemical arrangement 40 is used, in which the conductive layer 36 works as a cathode. A source of external voltage is applied, indicated as conventional in Figs 3 A-D. By exposing the membranes 20 to an electrolyte 37 and by branching an anode 38 at the side facing the open pores (here the upper side 25), electrodeposition occurs. During metallization, the nanopores 21 are preferably filled completely, filling up the end-to-end holes 21. Figs 3 B, C and D show how the nanowires 22 grow as far as out of the pores 21 on top of upper surface 25 and finally forming a continuous layer 39 across a plurality of pores 21. Preferably, a mask (not shown) is placed on the membrane 20 on the side of the anode 38, so as to limit metallization on the surface 25 of the membrane 20 to the position of the MEA electrode to be created. In principle, all conductive materials that can be deposited by electrodeposition may be used. In some embodiments, copper, gold, nickel and alloys containing one or more of these metals may be used, for example.

**Figure 4** shows the membrane 20 obtained in the process depicted above, from which the mask on the top has been removed. The membrane 20 contains electrodes M.1 and P.1, for example, each formed on top of a bundle of nanowires 22. The top end of the nanowires 22, emerging from upper surface 25, forms the array electrodes M. 1, P.1, and so forth, of the MEA in accordance with the embodiment shown. On the bottom surface, the continuous layer 36 will later form the electric contact with a circuit board (not shown). It is noted here that layer 36 is "continuous" in that it continuously covers, on the bottom surface 26 of the membrane 20, essentially all nanowires 22 that are associated with one particular array electrode. Layer 36 does generally not cover the entire surface 36 and does not span over different array electrodes. On the top surface, the continuous layer 39 will form an array electrode M.1, P.1, etc. of a MEA, preferably on the bottom of a sample-well, the well being adapted to receive living cells, tissue samples, or liquids to be tested in general.

The membrane of the MEA comprises groups of end-to-end nanoholes containing conductive nanolines or nanowires. As is apparent from the present specification, the conductive nanolines of one group of end-to-end nanoholes form one group of conductive nanolines. One group of conductive nanolines electrically connects one single array electrode (M. 1 or P. 1, ...) of said MEA to said circuit on the lower surface of said membrane. A group of nanolines comprises or consists of a plurality of nanolines.

One single or individual array electrode of said MEA is formed by or comprises a group of nanowires, which group of nanowires electrically connects the single array electrode (e.g. M.1) of said MEA to said circuit on the lower surface 26 of said membrane 20.

Preferably, one group of nanolines form and/or merge to one array electrode of the MEA, as illustrated, for example in Fig. 4. Since the MEA comprises a plurality of array electrodes, a MEA comprises a plurality of electrically separate groups of nanolines. The "plurality" generally refers to the number of array electrodes.

The expressions "one array electrode", "a single array electrode", "one single array electrode" and "one individual array electrode" are intended to refer to the characteristics of one specific array electrode of the MEA of the device of the invention, understanding that several, most or all of the other array electrodes have the same structure. The expression is not to be understood as "only one". The interpretation "only one" could erroneously be understood as meaning that (only) one array electrode of the MEA comprises a group of nanowires, while the other array electrodes do not, which is not the intended meaning of the expressions.

In the schematic Figure 4, four nanolines of array electrode M.1 are visible, belonging to the group of nanolines of array electrode M.1, whereas three nanolines 22 are visible with respect to array electrode P.1.

Figs 3-6 are schematic figures. A single array electrode, such M.1, comprises more than 1'000 nanowires. In an embodiment, one group of conductive lines comprises more than 4000, preferably more than 10⁴, even more preferably more than 2×10⁴ and most preferably more than 10⁵ and even more than 3×10⁵ conductive nanolines. One array electrode may comprise up to millions of nanowires. Within one array electrode (M.1 or P.1, for example) comprising a group of nanowires, the density of nanowires may be in the range of 10⁸ to 10¹¹ nanowires per cm². Preferably, within one array electrode, the density of nanowires may be in the range of 10⁹ to 10¹⁰ nanowires per cm².

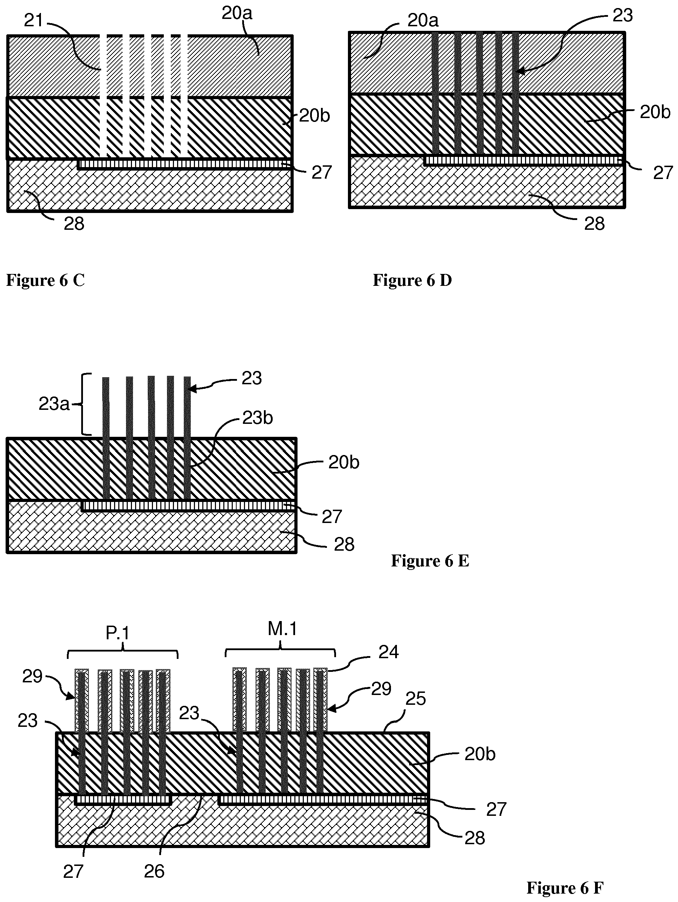

In an embodiment, the nanowires of a group of nanowires 22 of one array electrode are electrically separated and thus isolated within the membrane 20. The nanowires of one group of nanowires are thus electrically connected at least on the bottom 36 of the membrane 20 and possibly also on the top 39 of an individual array electrode. In the embodiments shown in Figs 6E and 6F, the nanowires 23 of a group of nanowires are electrically connected on the bottom only of the membrane 20b, via the electric circuit 28 but not on the top of the membrane.

In a preferred embodiment, each array electrode of the MEA comprises a group of nanowires. The present invention does not exclude the possibility of different array electrodes, for example a combination of array electrodes as described in the present specification and conventional array electrodes.

The skilled person will note that the same mask 30 (or the same type of mask) shown in Fig. 1 can be used repeatedly in different process steps resulting in the membrane 20 with the electrodes connected to a circuit board, in particular a PCB, thereby forming a MEA in accordance with preferred embodiments of the present invention. First, the membrane 30 is used for the ion-tracking step (Fig. 1), determining the position of the nanopores 21 and the future position of the nanowire-based array electrodes M.1, P.1 on the membrane 20. The mask 30 is again used for depositing the bottom contact layer 36 of the membrane 20, for example by PVD. The mask 30 can also be used for the step of growing the nanowires 22 and electrodes M.1, P.1 by electrodeposition (Fig. 3 A- D). Finally, the mask 30 is used when applying folder paste for attaching the membrane 20 with the nanowires 22 onto a circuit board, thereby electrically connecting the nanowire electrodes to the circuit board.

The membrane 20 may be connected to a circuit board as is conventional. In particular, the mask 30 that has been used in the step of ion tracking described with respect to Fig. 1 can be put onto a PCB for locally applying a conductive solder paste on the appropriate positions of electric contacts of the PCB. When the mask is removed, the membrane 20 with the nanowires 22 and array electrodes M.1, P.1 etc. may be placed onto the PCB so that the bottom contacts 36 (Fig. 4) come to lie onto the contacts containing the solder paste. In one or more further steps, the membrane 20 is pressed on the PCB and heated, thereby connecting the membrane 20 to the PCB and creating stable electric contacts between the electrodes and the electric contacts on the PCB. The electric contacts on the PCB are connected via electric lines on the PCB to electronic components such as switches, for example, as will be described in more detail below.

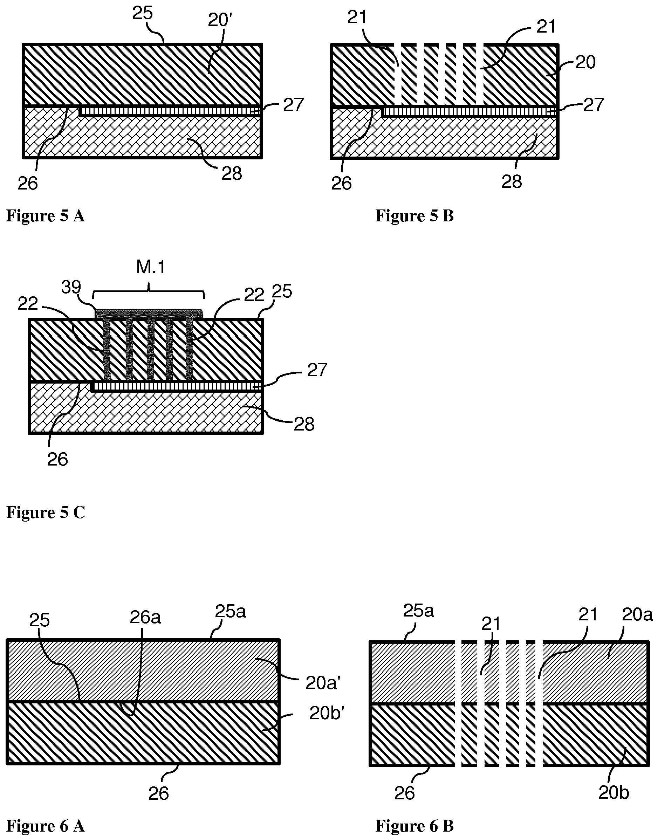

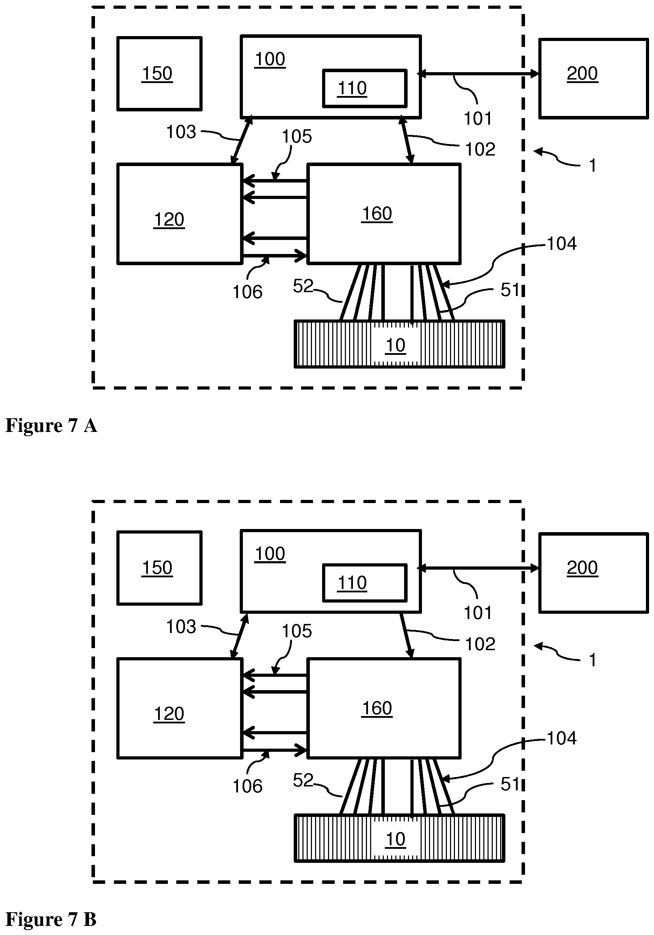

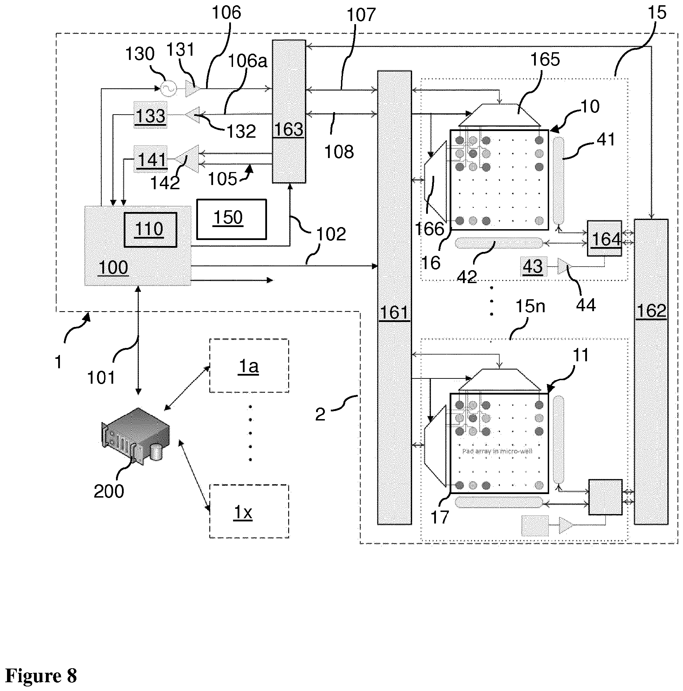

The fabrication of the MEA of the device of the invention can also be adapted to industrial production. For example, **Figures 5 A- C** illustrate a process in which the MEA is directly produced on a circuit board, preferably a PCB. In accordance with this method, as shown in Fig. 1 A, a precursor membrane 20' is attached to a circuit board 28, which contains conducting lines 27 at appropriate positions. The circuit board 28 was prepared in advance, to match the MEA to be prepared and preferably to fit the electronic configuration of the entire device containing the MEA (Figs 7 and 8). In Fig. 5 A, the membrane 20' has not yet be exposed to ion tracking. The entity comprising the precursor membrane 20' and the circuit board 28 is exposed to ion tracking and etching following the same principle as illustrated in Figs. 1 A and 1 B, using the appropriate mask (not shown). Fig. 5 B shows the circuit board 28 with the membrane containing nanopores 21 produced in the membrane 20 only.

The nanoholes 21 formed in the membrane 20 do not extend into or through the circuit board 28 and/or the circuit line 27, which is because the material of the circuit board was selected so as not to be sensitive to the ionic radiation and to the etching liquid. However, as the embodiments shown in Figs 1-3, end-to-end holes 21 crossing the entire membrane 20 are formed.

The production of nanowires by electrodeposition is illustrated in Fig. 5 C. In particular, the conducting circuit lines 27 are used as part of the electrochemical arrangement for metallization of the nanopores 21 and forming the nanowires 22. The conducting lines 27 at the lower surface 26 of the membrane 20 form the bottom of the pores and, at the bottom of the pores, constitute the cathode of the electrochemical deposition process, following otherwise the same principle as illustrated in Figs 3A-D.

In accordance with an embodiment, conductive circuit lines 27 provided on the circuit board provide the cathode and/or the origin of the metallization of the nanopores 21 and forming the nanowires 22.

In an embodiment, the electrodes M.1, P.1, etc. are not formed by a continuous layer 39, but are formed by a bundle of nano-hair or hair nano-stubbles formed by a plurality of nanowires 22 extending above the surface 25, as illustrated in Figs 6 E and F. In an embodiment, one or more array electrode M.1, P.1, etc. of said MEA 10 comprises a plurality of freestanding nanowires or stubbles 23a, 29. Preferably, said plurality of freestanding nanowires form one single array electrode M.1 or P.1, etc. In this case, the plurality (or group) of nanowires 22 of a single overall array electrode (for example, of electrode M.1) may generally not be connected on the top surface 25, but are connected as shown in Fig. 4 by a layer 36 at the bottom surface 26 of the membrane 20, or by conducting lines 27 of the circuit board (Figs 6 E and F). In accordance with this embodiment, each electrodes M.1, P.1, etc. has the form of a small brush of nanowires 23, 29 on the upper surface 25. Preferably, said free standing nanowires or stubbles 23a, 29 emerge from the top surface 25 of the membrane 20, 20b. In accordance with this embodiment, the upper surface of an array electrode may be nanostructured.

**Figures 6** **A** through **6 F** illustrate an exemplary process for obtaining a MEA with array electrodes in the form of a brush of nanowires or stubbles. As illustrated in Fig. 6 A, two precursor membranes 20a' and 20b' are attached one with respect to the other. In particular, one of the two flat, opposing sides of a membrane is attached to the flat side of the other membrane. In Fig. 6 A, the lower side or face 26a of the first precursor membrane 20a' is fixed on the upper side 25 of the second precursor membrane 20b'. The terms "upper" and "lower" do mean that the two opposing sides of the precursor membrane are necessarily different from the beginning. The distinction between "upper" and "lower" is used in this specification for better illustrating the process of fabrication of the array electrodes and for the purpose of convenience. It is noted, however, that the upper side 25 of the second precursor membrane 20b' is intended to form the bottom of the well containing the MEA. In this regard, in the operating device comprising the MEA shown in Fig. 6 F, the upper side 25 is generally expected to be vertically above the lower side 26.

The first and second precursor membranes, 20a' and 20b', are made from different materials having different chemical properties and/or different stability with respect to etching liquids, for example. For example, the first precursor membrane 20a' may be made from polycarbonate and the second precursor membrane 20b' from polyimide. The two precursor membranes 20a' and 20b' may have independently, a thickness as indicated elsewhere in this specification, for example in the range of 20-50 µm.

The superposed, precursor membranes 20a and 20b fixed on to the other are exposed to ion track-etching as described herein above with respect to Figs 1A and B, resulting in the nanopores 21 extending substantially from the top surface 25a of the first membrane 20a across both membranes to the bottom surface 26 of the second membrane 20b, as shown in Fig. 6 B. The nanoporous membrane assembly 20a, 20b of Fig. 6 B is then applied at the appropriate position on a circuit board 28, preferably a PCB, as shown in Fig. 6 C. The circuit board 28 has been produced previously to be adapted to receive the membrane assembly 20a, 20b, so that nanopores 21 of come to lie above appropriate circuit lines 27.

Fig. 6 D illustrates the metallization of the nanopores 21 so as to produce nanowires 23 across both membranes 20a, 20b. Accordingly, the circuit lines 27 on the circuit board 28 form cathodes at the bottom of the nanopores 21, where the nanopores meet the circuit lines 27 (Fig. 6 C). The assembly shown in Fig. 6 D, containing the circuit board 28, the membrane assembly 20a, 20b containing nanowires filled with nanowires 23, is exposed to a particular etching liquid, which specifically dissolves the first membrane 20a. The material of the second membrane 20b, however, was selected to be substantially inert to the etching liquid, so as to achieve selective dissolution of membrane 20a only, resulting in the assembly shown in Fig. 6 E. The nanowires 23 have two portions, a first or upper portion 23a, which corresponds to the part of the nanowires where they were located in the nanopores of the first membrane (Fig. 6 D), and the second portion 23b of the nanowires 23, which is the portion inside the second membrane 20b. The nanowires 23 thus extend in the pores of the second membrane 20b and extend above the upper surface 25 of the latter to be free standing, exposed to the air, forming "stubbles" emerging from the top surface 25 of the second membrane 20b. In an embodiment, said free standing nanowires or stubbles 23a, 29 are or comprise extensions of said conductive lines 23.

In an optional step illustrated in Fig. 6 F, the stubbles formed by the upper portion 23a of the nanowires 23 (Fig. 6 E) are coated in a subsequent step with a suitable metal 24, for example copper or gold, preferably by electrochemical deposition, so as to form coated nanostubbles 29 coated with a coating 24.

In this embodiment, a particular array electrode, such as M.1 or P.1, for example, is formed by a brush of nanowires instead of a dot or otherwise substantially uniform, homogenous substance of matter. Preferably, in a MEA, all array electrodes of a particular MEA and/or well are formed by a brush of freestanding nanowires or stubbles.

An advantage of the nanostubbles or free-standing wires is to measure directly the intracellular potential and/or impedance of cells. It is expected that the stubbles, due to their particular dimensions, are appropriate to penetrate the cell membrane of cells and thus allow measuring the potential across the membrane, for example.

In an embodiment, said electronic circuit with which the membrane is in contact is provided on an electronic circuit support board, and wherein said membrane is attached on said board so as to electrically connect each array electrode to said circuit board via said conductive lines in said plurality or group of holes.

The circuit board containing the electronic circuit is preferably substantially flat, so as to provide a substantially even support for the membrane containing the MEA.

As the skilled person will understand, Figures 1, 3 A-D, 4, 5 A-C and 6 A-F, in which the membrane (20, 20a, 20b, as applicable) and the circuit board (Figs 6 A-F) are schematically shown in cross section, are not true to scale. In particular in Figs 6 A-F, the membranes are shown much thicker compared to the circuit board, although the circuit board (generally a PCB) is in reality generally substantially thicker than the membrane. Exemplary thicknesses of membranes and circuit boards are given elsewhere in this specification.

In an embodiment, the membranes 20 containing the nanowires 22 is used as a MEA for biochips. In particular, the membranes containing the nanowires 22 may be used at the bottom of wells adapted to receive fluids, in particular fluids with cells, tissues, and so forth. In this case, one surface of the membrane 20, for example the lower surface 26, is preferably connected to a circuit plate containing electronic circuit lines, for example a printed circuit plate (PCB) (not shown). The other surface, for example the upper surface 25, forms the bottom of a well. Generally, the well receiving the sample of liquid and/or containing cells is a micro-well. Accordingly, the fluid and thus the cells will be exposed to the electrodes M.1, P.1, etc. of Fig. 4, for example, or to the nanobrush-electrodes as described above.

In an embodiment, the MEA comprises 4 or more, preferably 8 or more, more preferably 16 or more, and most preferably 32 or more array electrodes M.1, P.1, etc. In an embodiment, the MEA comprises 40 or more, for example 50 or more and in other examples 60 or more array electrodes. For example, the MEA 10 shown in Fig. 8 has 49 array electrodes. It is noted that one array electrode is an electrode that is electrically separate from other array electrodes and/or an electrode that differentiates over other array electrodes in that it can be individually activated or targeted for taking measurements.

The "well" preferably comprises at least a bottom surface and side walls forming together a receptacle for receiving the culture medium and/or the sample. The well may also contain a lid on its top, which may be opened for adding, culture medium and/or cells, or any kind of liquid to be analyzed. The side walls may be essentially cylindrical, forming a continuous side wall, or may be separate side walls joined to form the receptacle. The well is generally adapted to receive a volume in the range of 1 µl to 2 ml, preferably 10 µl to 1 ml, more preferably 100 µl to 500 µl.

**Figures 7** **A** and **B** illustrate an embodiment of a portable device 1 for assisting the operation of a MEA. Preferably, the MEA is as described herein above and elsewhere in this specification.

In an embodiment, the device 1, of the invention is a hand-held or a hand-holdable device. The device is preferably separate from a computer 200. Preferably, the computer 200 contains and/or is configured to run software for operating the device.

The portable device 1 may comprise the elements within the box with the dotted contour in Figs. 7 A and B. In particular, the portable device comprises an on-device controller 100, configured to communicate with a larger data processing system 200, for example a server 200. The exchange of data between the on-device controller 100 and the server may take place by cable or, in a preferred embodiment, by a wireless connection, both possibilities being indicated by reference numeral 101 in Fig. 7 A. The emitter and receiver, or more generally, a wireless communication module, in case of a wireless connection are also present on the portable device 1, but are not explicitly shown. If the connection 101 is wireless, a portable power source 150 has to be present on the device, for example a battery, which may be rechargeable and/or replaceable. The portable power source 150 may also be a separate battery that is connected by cable to the device 1, but which can be displaced with the device 1. In contrast, in embodiments where the connection 101 to the server 200 is a cable, the cable may also provide electric energy for running the portable device 1 and the power source 150 may not be necessary.

In an embodiment, the device of the invention comprises a battery 150, wherein said controller 100 is configured to communicate with said computer 200 via wireless data transmission, so that said device can be displaced independently/without physical connection to said computer 200. Preferably, said battery is a rechargeable battery.

The controller 100 is configured to communicate with a switch and/or selector assembly or unit 160 and with a measuring assembly or unit 120, for example via lines 102 and 103, respectively. The switch assembly 160 is configured to open circuit lines in accordance with instructions received from the controller 100. Preferably, a plurality of circuit lines 104 connect the switch assembly 160 with the electrodes of the MEA 10, wherein each electrode contains one individual line by which it is connected to the switch assembly. It is noted that while the switch assembly is shown as a unit 160, it may comprise a plurality of separate switches, as will be discussed further below with respect to preferred embodiments of the present invention.

The controller 100 comprises and/or controls a local storage unit 110, which is preferably a temporary storage 110. In an embodiment, the controller is adapted and/or configured to compress data received from said measuring subassembly 140, so as to forward compressed data to said computer 200 via said wireless data transmission or via said cable.

The measuring unit 120 is adapted to perform measurements related to the electric characteristics of the sample in the MEA 10, via the switch assembly 160. For example, the measuring unit 120 may be configured to measure impedance between two array electrodes of the MEA or may be configured to measure a potential difference between two array electrodes, or may be equipped to measure both, independently, for example. Of course, the measuring unit may also determine impedance between and/or a potential difference between an array electrode and a wall electrode.

As the skilled person will understand, variation in complex impedance in a biological sample is associated with the presence of living cells, the latter colonizing the bottom surfaces of the wells and thereby the array electrodes of the MEA. On the other hand, the potential differences are useful to detect activity of cells producing potential differences, such as action potentials and the like. In particular, nerve cells, muscle cells (for example, myocardial muscle cells) and endocrine cells rely on electric signals for their activity. By measuring potential differences between electrodes, activities of cells on or near the electrodes, such activity may be detected and monitored, for example.

In a preferred embodiment, the device of the invention comprises a controller 100, configured to communicate with a computer 200 via wireless data transmission or via a cable, wherein said device 1 further comprises a one or more switches or selectors 160 that can be activated by said controller 100 for selecting electrodes of said MEA for the purpose of conducting measurements of electric characteristics of a sample in said well, wherein said device further comprises a measuring subassembly 140 for measuring said electric characteristics.

The functioning of the device of Fig. 7 A is now illustrated with reference to Fig. 7 B. The controller 100 is configured to control the taking of measurements from the sample placed in the well comprising MEA 10. The controller 100 thus causes switch assembly 160 to open a particular set of circuit lines, for example lines 51 and 52 of two electrodes of the MEA 10. Generally, measurements are taken between two neighboring electrodes of the MEA. Depending on the measurement to be taken, the switch assembly opens circuit lines 105 or 106 to the measuring unit 120. In the example shown in Fig. 7 B, circuit lines 106 are opened, which allow measurement of impedance between the two electrodes opened by switch assembly 160. Via line 103, the controller 100 has sent information to the measuring unit 120 instructing it to measure impedance and receive the corresponding signal related to impedance back from the measuring unit via a dedicated line, for example (also illustrated by double headed arrow 103). The information related to the measurement received from the measuring assembly 120 may be stored on the local storage 110. A series of measurements is generally taken from pairs of electrodes of the MEA, with one measurement taken between two electrodes of a particular MEA at a time, followed by the next measurement taken between another pair of electrodes, and so forth, until a determined number of measurements between different pairs of electrodes are taken. It is also possible to use a given electrode in different pairs of electrodes, for example a measurement between the pair M.1 and P.1 and another measurement between the pair M. 1 and P.2, for example.

Which pair of electrodes is selected for taking measurements and which type of measurement is taken is determined by the software on the system 200 and/or on the controller 100. It is also possible to take measurements from a single electrode, for example a wall electrode, with respect to a reference electrode, the latter not necessarily being part of the MEA but being exposed to the liquid of the sample in the MEA.

If the device comprises a plurality of MEAs, for example 10, 11, etc., (Fig. 8), the invention encompasses the taking of measurements simultaneously from different MEAs.

The device 1 is preferably a portable and/or displaceable device. Therefore, it can be moved and/or displaced conveniently, even with a sample of cells contained on the MEA, and/or in the well containing the MEA. If the connection 101 is by cable, the cable is preferably flexible and has sufficient length for allowing displacement of the device 1 within the area defined by the length of cable 101. If the connection 101 is wireless, the freedom of moving the device may be determined by the reach of the wireless system 101 of device 1 and server 200. It is not excluded that server 200 and device 1 are connected via the internet, so that the location of the server and device 1 need not be in the same room, or building, but the server 200 and the device 1 may be in different rooms, different buildings, or separated by long distances (> 1 km, for example). A wireless connection 101 is preferred, because the latter allows the portable device 1 to be placed in an environment that is conducive to cellular growth or which simulates physiological conditions. In an embodiment, the device of the invention is operational inside an incubator and/or is operational at conditions that are conducive to the cultivation of living cells. For example, the portable device 1 can be placed inside an incubator (not shown) wherein the server 200 remains outside the incubator. The device may thus be adapted to support the temperature (for example 20-40°C, preferably 30°-39°) and humidity (preferably 50-100% relative humidity RH, more preferably 60-100% RH, most preferably 70-100%RH) inside the incubator, whereas the server 200 does not need to be adapted to support such conditions. The conditions are preferably chosen so as to avoid and/or reduce evaporation of the sample in the well of the MEA 10.

In a preferred embodiment, the MEA 10 is removable and/or detachably placed on the device 1. In an embodiment, the device comprises one or more a detachable entities, wherein each entity comprises preferably at least the MEA. In an embodiment, the detachable entity may comprise the well (including the lateral wall of the well) and the MEA contained in the well. Generally, the MEA is on the bottom of the well. In yet another embodiment, the detachable entity comprises the MEA, the well and possibly one or more switches that are electrically connected directly to the MEA. In terms of reduction of waste, however, the smaller the detachable unit is, the better. In an advantageous embodiment, the MEA can be removed alone, in particular the MEA comprising the membrane 20 and the electrodes (M.1, P.1, etc) on top of the membrane. In another embodiment, the MEA 10 including the membrane 20 and the circuit board on which it is fixed (not shown) as described above forms the removable entity. In a preferred embodiment, the removable entity comprises the MEA and the well but preferably lacks array electrode switches.

It is a particular advantage of the invention that the MEA can be replaced after use as part of the detachable entity. This allows many electronic components of the device 1 to be used several times, for different experiments, whereas the MEA, when used once, can be discarded. In this way, the detachable unit containing the MEA is a consumable, which is limited to a large extent to the part that is exposed to a sample. In another embodiment, the detachable unit comprising the MEA is adapted to support washing and/or autoclaving and is thus reusable.

In an embodiment, the detachable unit is attached via a detachable click connection onto the device 1. In another embodiment, the detachable unit is fixed by way of one or more screws that is/are easily accessible by a user and/or operator of the portable device 1. The present invention encompasses all types of removable and/or detachable connections for replaceably connecting the detachable unit onto the device 1. Such connections may comprise locks or security assemblies for avoiding inadvertent detachment of the detachable unit comprising the MEA from the device 1. Preferably, the device 1 and/or the detachable unit comprise complementary geometric arrangements for guiding the correct connection of the detachable on the device. In particular, with the detachable unit being connected, the MEA 10 is preferably automatically connected correctly to the circuit lines 104, for example 51, 52, etc, so that the device 1 on which the detachable unit is fixed is automatically operational. Preferably, the device is adapted, for example by way of complementary geometric arrangements fitting together (not shown), to avoid incorrect connection of the detachable unit to the portable device 1.

Fig. 8 shows a device 1 comprising a plurality of sample wells and, accordingly, a plurality of MEAs. In an embodiment, the device 1 of the invention comprises a plurality of MEAs 10, 11 and/or a plurality of wells 16, 17, wherein said one or more switches 160 comprises a MEA selector switch 161, which selects a specific MEA 10 of said plurality of MEAs for conducting a measurement in the well 16 of said specific MEA. In an embodiment, the device 1 comprises a plurality of separate detachable units, each detachable unit comprising a MEA.

In an embodiment, the controller 100 is configured to preprocess and/or compress data. Accordingly, the controller is configured to differentiate between useful and useless data. In an embodiment, the controller is configured to determine electrodes or pairs of electrodes from which measurements are interesting from those electrodes, where there is no activity or where there are no living cells. In this manner, the controller 100 transmits only relevant data to the server 200. Thanks to the data compression and/or selection, the quantity of data to be transmitted from the controller to the server 200 can be reduced to a minimum.

When measuring nervous cell potentials, for example, full-time sampling is required, which creates massive amounts of data, especially when there are many wells with many electrodes. The present invention provides data compression. For example, the controller 100 contains and/or is configured to run a program which analyses the measured data and applies one or more compression algorithms. A compression algorithm may be based on pattern matching, for example when measuring nervous action potentials. An action potential can generally be defined accurately by a few (for example, four to ten) specific data points. Data may also be compressed by "lossy compression", for example. Thanks to the compression of data, a less performing data transfer line may be used. For example, the compressed data can be forwarded to the server 200, by a low data rate channel, preferably by wireless data transfer.

In an embodiment, the said controller 100 comprises a temporary storage 110 and is adapted to compress data received from said measuring subassembly 140, so as to forward compressed data to said computer 200 via said cable or via said wireless data transmission.

**Figure 8** shows in more detail a portable device 1 according to a preferred embodiment of the invention, in which the same reference numerals refer to the same components as in Figs. 5 A and B. The device 1 preferably comprises a support 2 on which or to which the components of the device 1 are attached. In the embodiment shown, the device 1 comprises two explicitly shown MEAs, a first MEA 10 and an n^{th} MEA 11, which may be a second MEA, for example. In some embodiments, the device 1 comprises more than two MEAs, for example more than 3 or more than 4 MEAs. In an embodiment, the device 1 comprises 1 to n MEAs, wherein n is an integer of 2-1536 or higher. Furthermore, the server 200 may be configured to communicate not only with the device 1, but with a plurality of devices, for example with a first device 1, a second device 1a and an x^{th} device 1x, as illustrated by dashed boxes 1a and 1x. In another embodiment, the server 200 may be configured to operate independently with more than three portable devices in accordance with the invention. In some embodiments, there are ≥ 1, preferably ≥ 2, more preferably ≥ 5, for example ≥ 10 different portable devices that can be controlled by system 200. The present invention envisages that up to 100 or even more devices can be controlled by data processing system 200.

The support 2 is preferably a multi-well plate containing a plurality of wells, each well containing a MEA.

The dashed box with reference numeral 15 designates the electronic components concerned specifically and only with the first well 16 containing MEA 10, whereas 15n designates the electronic components specifically concerned with the n^{th} well 17. Herein after, only the electronic components of the first well 16 will be discussed, as all n wells preferably comprise substantially identical components and/or structure. In the embodiment shown, the first well or MEA subsystem 15 comprises: the first well 16 comprising the first MEA 10, but also two wall or reference electrodes 41 and 42, as well as a reference voltage generator 43 and current buffer amplifier 44. The switch 164 is adapted to open circuit lines as desired to the wall electrodes 41, 42 and the reference electrode 43.

The wall electrodes 41, 42 may be used, for example, to set the electric potential of the liquid in the sample well. The liquid is generally a medium for the cells that are cultivated in the liquid. Generally, the reference voltage produced by reference voltage generator 43 and current buffer amplifier 44 are used to determine the electric potential of the liquid in the well. Any suitable reference voltage may be produced by the reference entity 43, 44. Typically, a voltage of 1.2-1.3 V may be set. The reference voltage may be used as the electrical zero point for subsystem 15.

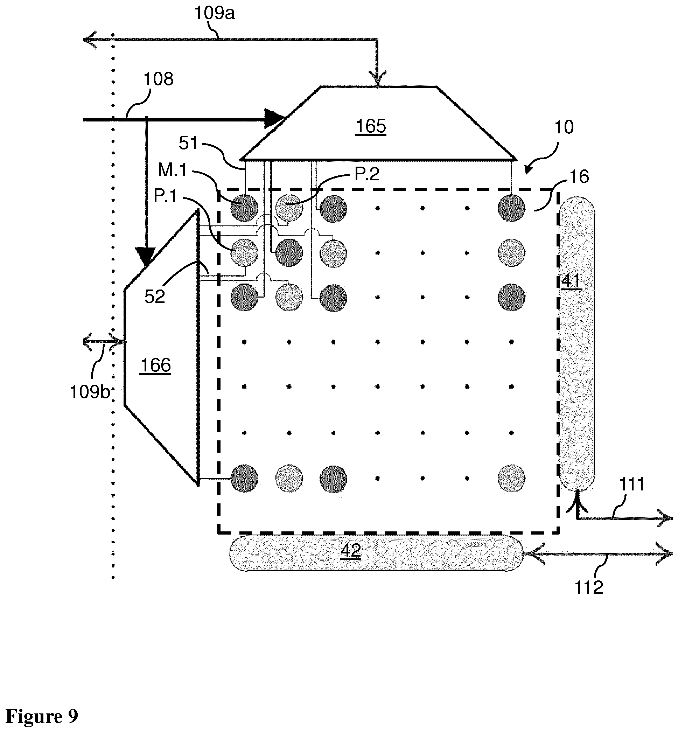

The array electrodes of the MEA 10 are selected by the switches 165 and 166, which can be seen as a first switch 165 and a second switch 166. These switches are array electrode switches, as they select the electrodes of the MEA. The configuration of the switches 165 and 166 and the array electrodes M.1, P.1, etc. can better be seen in Fig. 9 and will thus be described in more detail below. It is clear that each switch 165, 166, is connected to a plurality of lines, each line leading to a particular array electrode (M.1, P.1, etc). In the embodiment shown there are two types of array electrodes, depending on the switch (first or second) that is connected to the electrode. Accordingly, in Fig. 8, dark colored dots represent array electrodes that are connected to the first switch 165 and light colored dots are connected to the second switch 166. Other MEA topologies than the one disclosed in Fig. 8 are discussed below.

The device 1 contains a plurality of further switches 161, 162, and 163, which assist in the operation of the device. Well selector 161 transmits instructions from the controller 100 to open selected lines of a pair (or plurality) of array switches of the very well from which a measurement is to be taken at a time. Accordingly, well selector 161 may open two lines to a particular well, for example well 16 or 17, etc. At the same time, well selector 161 opens lines 107 and 108 to the measurement selector 163, to allow an appropriate measurement as determined by the controller 100. The measurement selector 163 opens lines in dependence of the measurement to be taken, such as in particular impedance or potential difference. Selector 162 is the selector of the wall or reference electrode of a particular well at a given time.

Regarding the measurements to be taken, the exemplary device 1 shown in Fig. 8 is adapted to measure impedance between two electrodes as well as the potential difference between two electrodes. Accordingly, the device 1 may comprise a subassembly for measuring impedance and a subassembly for measuring potential difference. In a preferred embodiment, the measuring assembly 140 comprises an impedance measuring unit 130-133 for measuring impedance between two electrodes of a MEA, and/or a potential difference measuring unit 141-142 for measuring the potential difference between two electrodes of a MEA.

Depending on the measurement to be taken, measurement selector opens a pair of lines 105 for measuring potential difference or a pair of lines 106 for measuring impedance.

The subassembly for measuring impedance 130, 131, 132 133 comprises an alternative current generator 130 and a current buffer amplifier 131 for sending a current via selector 163 to a MEA electrode. The AC generator may produce an AC voltage having a frequency in the range of 1kHz to 1MHz, for example. The AC generator 130 and the buffer 131 produce a voltage to which a determined MEA electrode will be exposed. For measuring impedance, a I→V current intensity to voltage circuit 132 and a complex impedance measurement unit 133 are provided.

The subassembly for measuring potential difference 141, 142 preferably comprises the potential difference amplifier 142 and the analog to digital converter 141. The amplifier 142 amplifies the potential difference that is measured between two MEA electrodes of a given well, and the signal may be sent via the converter 141 to the controller 100 for further treatment and/or temporary storage.

In an embodiment, the device of the invention comprises a plurality of switches 160 comprising, for each MEA, at least two separate array electrode selectors, a first array electrode selector 165 and a second array electrode selector 166, wherein said plurality of array electrodes comprise a first group of n array electrodes (M.1, M.2, ..., M.n) and a second group of m array electrodes (P.1, P.2, ..., P.m), wherein each electrode of said first group of electrodes comprises a separate and/or own circuit line to said first selector 165, and each electrode of said second group of electrodes comprises a separate and/or own circuit line to said second selector 166, and wherein said controller 100 is configured to conduct a measurement between any selected electrode (M.x) of said first group and any selected electrode (P.y) of said second group.

The working principle of the device of the invention is set out at the example of measuring impedance. Controller 100 sends signals to the switches 161, 163 and to the impedance measurement assembly 130-133. The signal sent to well selector 161 contains also information with respect to the array electrodes that are to be addressed. For example, the signal passes through switch 161 to array switches 165 and 166, which each open a line to an electrode, preferably such that lines 51, 52 (Fig. 9) to two neighboring electrodes, M.1 and M.1 are opened by switches 165 and 166, respectively. In addition, the lines 107 and 108 between switches 161 and 163 are opened by switch 161. Measurement selector 163 is instructed to open the pair of line 106 and 106a, for allowing a measurement of impedance. In this illustrative example, the switches 161, 163, 165 and 166 created two, electrically separated, continuously open circuit lines. The first line extends from the AC generator 130 (or buffer 131) to electrode M.1 of MEA 10, via lines 106, 107, 109a, 51 (Fig. 9). The second open line extends from the complex impedance measurement unit 133 (or from the I→V circuit) to electrode P.1, via lines 106a, 108, 109b and 52. The AC generator is instructed to produce an alternate current at a set frequency, which is transmitted over the first open line to the array electrode M.1. Impedance between electrodes M.1 and P.1 can now be measured at the complex impedance measurement unit, which transmits the measured value to the controller.

Practically, measurements of impedance are preferably done in a swept-frequency mode, such that a plurality of measurements are taken across a frequency spectrum, for example between 1kH and 1 MHz, with respect to a particular pair of electrodes, for example a pair of array electrodes. In other words, impedance is measured by scanning a pair of electrodes across a range of frequencies and measuring impedance for each specific frequency of the frequency scan.

From the measurements taken from pair of electrodes, the controller may calculate particular circuit equivalents with respect to a pair of electrodes, for example between a pair of array electrodes. The circuit equivalents characterize the state of the cells in the sample. Typically, circuit equivalents are resistivity and capacities related to the impedance measurements.

The device of the invention allows measurement of an electric characteristic between any pair of array electrodes of a particular MEA, as determined by the controller. The controller, in turn, is configured by appropriate software to conduct measurements as determined by the operator. For measuring potential difference between array electrodes, switch 163 opens the pair of lines 105 and keeps lines 106 and 106a closed, and the potential difference is measured at the converter 141, which transmits the measured value to the controller 100. Lines to particular pairs of array electrodes are opened by switches 161, and, with respect to MEA 15, switches 165 and 166 as described above.

In an embodiment, the device 1 of the invention comprises a measuring assembly 120 for measuring impedance 130-133 but lacks a measuring assembly for measuring the potential difference 141, 142. In another embodiment, the device 1 of the invention comprises a measuring assembly 120 for measuring the potential difference 141, 142 but lacks a measuring assembly for measuring impedance 130-133.

The device 1 of Fig. 8 preferably comprises a local source of electric energy 150, preferably a battery, for example a rechargeable battery.

Although not specifically shown, the device 1 of the invention may comprise further measuring entities or assemblies, such as a subassembly for measuring pH of the liquid in the sample well, salt concentration, for example metal ion concentration, and/or any additional electric measurement.

**Figure 9** is an extract of Fig. 8, showing in more detail a MEA of a preferred embodiment. The dotted box 10 defines the MEA with its array of electrodes, such as electrodes M.1, P.1, etc. From each array electrode, a circuit line is drawn to the respective switch 165 or 166 enabling access to that specific array electrode. By sending instructions via well selector 161 to the switches 165 and 166 (line 108), open circuit lines leading to two electrodes M.a and P.b are opened, wherein M.a is an electrode of selector 165 and electrode P.b is an electrode of selector 166. Preferably, M.a and P.b are neighboring electrodes or electrodes that are not too distanced, such as M.1 and P.1. The opened circuit lines to two array electrodes M.1 and P.1 allow measurement via circuit lines 51 and 52, leading from the respective array electrode selector 165, 166 to the respective measurement assembly. In order to do so, well selector 161 and measurement selector 163 are adjusted to open a complete circuit line to the respective measurement unit for measuring impedance or potential difference, as determined by the controller 100.

In Fig. 9, the sample well is illustrated by the box 16 with the dotted lines. The well contains walls so as to confine the sample on the MEA. The wall electrodes 41, 42 may be integrated to the walls or fixed to the walls of the sample well.

As has been mentioned above, the MEA 10 is part of a detachable entity, which can be removed from the device 1 and in particular from the support 2 of the detachable and/or disposable entity, for replacement by a new, unused MEA. In an embodiment, the well 16 comprising said MEA 10 are part of the disposable replacement unit, wherein said replacement unit is detachably mounted on said device 1 so as to be detached from said device after use.

In an embodiment, the well 16 comprising said MEA 10 form a single-use entity.

The one or more wall electrodes may or may not be part of the detachable entity. Generally, the wall electrodes are rigidly connected to the inside of the walls of the sample well, so as to be in contact with the liquid of the sample. Accordingly, when the sample well is part of the detachable entity, the one or more wall electrodes are also part of the detachable entity. The wall electrodes may thus be part of the single use entity of the device. Of course, it is preferred to detach as little as necessary after use of the MEA biochip and to reuse a maximum of components. Therefore, in a preferred embodiment, the array electrode selectors 165, 166 are preferably not part of the detachable entity. In other embodiments, the array electrode switches are also detached as part of the detachable entity, which is generally disposed after single use, as described elsewhere in this specification. In other words, the device 1, with exception of the detachable, single-use entity comprising at least a well and the MEA, is adapted for repeated use and/or reuse. Accordingly, the device is intended and adapted to multi-use. The device as such is not disposable and/or is not a single-use device.

In a preferred embodiment, the device of the invention comprises one or more detachable single use entity and multi-use components, wherein said well 16 comprising said MEA 10 are part of the single use entity, and wherein said single use entity is detachably mounted on said device 1 so as to be detached from said device after a single use. The multi-use components are preferably configured to be equipped with a new, exchangeable single-use entity. In particular, the controller 100 and the measuring unit 120, 133, 141, 142 are preferably multiuse components. The battery 150, if present, is preferably also a multiuse component. Wall electrodes 41, 42 may be part of the single-use entity or may be part of the multiuse components. The support 2 is preferably a multiuse component in as far as multiuse components are fixed on it.

In an embodiment, the one or more switches 160, preferably all switches, are multiuse components and thus not part of the single-use unit. In an embodiment, one or more of the selectors are multi-use and thus not detachably mounted on the device, while one or more other selectors, for example the array electrode selectors 165, 166, are part of the single-use entity.

The detachable elements and/or the detachable entity of the device of the invention are preferably designed for single use. Preferably, the detachable elements can be replaced. In an embodiment, the detachable elements comprise at least the MEA, preferably the entire well containing the MEA, and more preferably also the electronic circuit (circuit board) below the MEA. The circuit board carrying and connected to the MEA may be referred to as a local and/or detachable circuit board. In some embodiments, the MEA is attached, for example bonded to a detachable circuit board, so that the circuit board has to be removed together with the MEA. The entity of detachable circuit board and MEA, extracts of which are shown, for example, in Figs. 5 C and 6 F, is preferably the detachable entity. In an embodiment, the switches for the array electrodes, such as switches 165 and 166, are detachable together with the MEA 10 and are also part of the detachable unit. The detachable elements can be replaced, so that the device 1 can be used as a whole for further experiments. The device 1 as a whole is thus preferably reusable. According to an embodiment, the MEAs of the device 1 comprises a circuit board that remains part of the device and another (detachable) circuit board on which the MEA is connected, which other circuit board can be removed from the remaining circuit of the device, in particular without damaging the integrity of the remaining electric circuit of the device. A fresh, unused disposable entity can be attached in place of the detached, used entity so as to connect properly to the (non-detachable) electric circuit of the device.

It is noted that Fig. 9 shows an embodiment with two array-electrode selectors or switches 165 and 166 per MEA. In this embodiment, the array electrode switches may be arranged on the two sides of the square MEA (Fig. 8, Fig. 9). In other embodiments, the device of the invention may comprise three or more array electrode switches per a single MEA. In an embodiment, the device comprises a first, second and third array electrode selectors. In this case, the array electrodes forming the MEA may preferably be arranged on a hexagonal grid as opposed to a square grid. In a hexagonal grid, any electrode preferably has six neighboring electrodes. With respect to the outline when seen from above, MEA may have a square, hexagonal or any other outline, independently from the grid used.

If the MEA has a hexagonal outline, the three array electrode switches may be arranged on three sides of the electrode array, for example.

A MEA with the electrode arrangement as shown in Figs. 8 and 9 provides four neighboring array electrodes that are under the control of another switch. In other words, a dark electrode in Fig. 9 (controlled by switch 165) has four bright electrode neighbors that are controlled by switch 166. This only applies for electrodes that are not provided along the side lines, the latter only having three neighbors. Array electrodes in the corners of the array have two neighbors. In a MEA having geometric electrode arrangement involving three or more array electrode selectors, each array electrode may have six or possibly eight neighbors controlled by another switch.

In an embodiment, an array electrode connected by a line to the first array electrode selector has at least three different electrode neighbors connected to the second array electrode switch and at least three different electrode neighbors connected to the third array electrode switch. In yet another embodiment, an array electrode connected by a line to the first array electrode selector comprises four electrode neighbors connected to the second array electrode switch and four electrode neighbors connected to the third array electrode switch. Accordingly, measurements could be made between an electrode and six or eight neighbors, in accordance with the respective embodiment. Such arrangements are in particular possible if each MEA has an own measuring assembly and possibly an own controller, as described below.

In an embodiment, each of the MEAs (10, 11, etc) of the device 1 has its own measuring assembly. In this case, there is no need to share the measuring assembly 120, such as the impedance measuring subassembly (130-133) and/or the potential difference measuring subassembly (141, 142) between the different MEAs. In an embodiment, each sample well has its own subassembly for measuring impedance. In this embodiment, each MEA and well may also have its own local controller for controlling the measurements of the respective well. While certain of the preferred embodiments of the present invention have been described and specifically exemplified above, it is not intended that the invention be limited to such embodiments. The invention is defined by the attached claims.

## Claims

1. A device (1) comprising one or more well (16) adapted to receive a sample with cells, wherein each well (16) comprises a multielectrode array (MEA) (10), wherein said MEA (10) comprises a membrane (20) on which a plurality of array electrodes (M.1, P.1, ...) are arranged to form the array of said MEA (10), wherein said membrane (20) comprises an upper surface (25) on which said array electrodes (M.1, P.1, ...) emerge, and a lower surface (26) where said membrane (20) is in contact with electronic circuit (27) connecting the array electrodes (M.1, P.1, ...) to electronic equipment assisting in the operation of the MEA, ***characterized in that*** the membrane (20) comprises groups of end-to-end nanoholes (21) containing conductive nanolines (23), and wherein one group of conductive nanolines (23) comprises more than 1000 nanolines and electrically connects one single array electrode (M.1 or P.1, ...) of said MEA to said circuit on the lower surface (26) of said membrane (20), and **in that** said electronic circuit (27) is provided on an electronic circuit support board (28), wherein said membrane (20) is attached on said board (28) so as to electrically connect an array electrode (M.1, P.1, ...) to said circuit board (28) via said conductive nanolines (23) in a group of nanoholes (21).

2. The device of claim 1, wherein one group of conductive nanolines (23) comprises more than 10'000, and most preferably more than 50'000 conductive nanolines and wherein the end-to-end holes (21) of a group of nanoholes have a mean diameter in the range of 10 nm to 5 µm, preferably 15-700 nm, more preferably 20- 300 nm, most preferably 30-100 nm.

3. The device of any one of the preceding claims, wherein said membrane comprises or consists essentially of a material selected from polycarbonate, polyimide, quartz, mica, glass, semiconductors and alumina.

4. The device of any one of the preceding claims, wherein one or more array electrode (M.1, P.1, ...) of said MEA (10) comprises a plurality of freestanding nanowires or stubbles (23a, 29), said plurality of freestanding nanowires forming one single array electrode (M.1 or P.1, ...).

5. The device of claim 4, wherein said free standing nanowires or stubbles (23a, 29) emerge from the top surface 25 of the membrane (20, 20b).

6. The device of claim 4 or claim 5, wherein said free standing nanowires or stubbles (23a, 29) are or comprise extensions of said conductive nanolines (23).

7. The device of any one of the preceding claims, which further comprises a controller (100), configured to communicate with a computer (200) via wireless data transmission or via a cable, wherein said device (1) further comprises a one or more switches or selectors (160) that can be activated by said controller (100) for selecting electrodes of said MEA for the purpose of conducting measurements of electric characteristics of a sample in said well, wherein said device further comprises a measuring subassembly (140) for measuring said electric characteristics.

8. The device of of any one of the preceding claims, which is a hand-held or a hand-holdable device and/or which is separate from a computer (200) comprising software for operating the device.

9. The device of claim 7, wherein said controller (100) comprises a temporary storage (110) and is adapted to compress data received from said measuring subassembly (140), so as to forward compressed data to said computer (200) via said wireless data transmission or via said cable.

10. The device of any one of claims 7-9, comprising a rechargeable battery (150) and a wireless communication module, wherein said controller (100) is configured to communicate with said computer (200) via wireless data transmission, such that said device can be displaced independently/without physical connection to said computer (200).

11. The device of any one of claims 7 to 10, which comprises a plurality of MEAs (10) and/or a plurality of wells (16), wherein said one or more switches (160) comprises a MEA selector switch (161), which selects a specific MEA (10) of said plurality of MEAs for conducting a measurement in the well (16) of said specific MEA.

12. The device of any one of the preceding claims, comprising one or more detachable single use entity (16, 10) and multi-use components (100, 120), wherein said well (16) comprising said MEA (10) are part of the single use entity, and wherein said single use entity is detachably mounted on said device (1) so as to be detached from said device after a single use.

13. The device of any one of claims 7-12, wherein said one or more switches (160) comprise, for each MEA, at least two separate array electrode selectors (165, 166), a first array electrode selector (165) and a second array electrode selector (166), wherein said plurality of array electrodes comprise a first group of n array electrodes (M.1, M.2, ..., M.n) and a second group of m array electrodes (P.1, P.2, ..., P.m), wherein each electrode of said first group of electrodes comprises a separate and/or own circuit line to said first selector (165), and each electrode of said second group of electrodes comprises a separate and/or own circuit line to said second selector (166), and wherein said controller (100) is configured to conduct a measurement between any selected electrode (M.x) of said first group and any selected electrode (P.y) of said second group.

14. The device of claim 1, wherein said MEA is produced by a method comprising the steps of:
(1) providing a precursor membrane (20') on a circuit board (28), the circuit board (28) containing conducting lines (27);
(2) producing nanopores on appropriate positions in the precursor membrane (20') while the precursor membrane (20') is attached to the circuit board (28), thereby obtaining a membrane (20) containing nanopores (21);
(3) producing nanolines inside said nanopores from the bottom of the nanopores to the upper surface (25) of the membrane; and,
(4) forming array electrodes of said MEA on the top surface of said MEA, thereby obtaining a MEA, wherein an individual array electrode of said MEA is formed by a group comprising more than 1'000 nanolines.

## Patentansprüche

1. Vorrichtung (1), umfassend eine oder mehrere Vertiefung(en) (16), die zum Aufnehmen einer Probe mit Zellen geeignet ist/sind, wobei jede Vertiefung (16) eine Multielektrodenanordnung (MEA) (10) umfasst, wobei jede MEA (10) eine Membran (20) umfasst, auf der eine Vielzahl von Anordnungselektroden (M.1, P.1, ...) angeordnet ist, um die Anordnung der MEA (10) zu bilden, wobei die genannte Membran (20) eine obere Oberfläche (25), auf der die genannten Anordnungselektroden (M.1, P.1, ...) zum Vorschein kommen, und eine untere Oberfläche (26), auf der die genannte Membran (20) mit einer elektronischen Schaltung (27) in Kontakt ist, die die Anordnungselektroden (M.1, P.1, ...) an elektronische Ausstattung anschließt, die den Betrieb der MEA unterstützt, umfasst, ***dadurch gekennzeichnet, dass*** die Membran (20) Gruppen von durchgehenden Nanolöchern (21) umfasst, die leitende Nanoleitungen (23) enthalten, und wobei eine Gruppe von leitenden Nanoleitungen (23) mehr als 1'000 Nanoleitungen umfasst und eine einzige Anordnungselektrode (M.1 oder P.1, ...) der genannten MEA an die genannte Schaltung auf der unteren Oberfläche (26) der genannten Membran (20) elektrisch anschließt, und dadurch, dass die genannte elektronische Schaltung (27) auf einer elektronischen Leiterstützplatte (28) bereitgestellt ist, wobei die genannte Membran (20) auf der genannten Platte (28) derart befestigt ist, dass sie elektrisch eine Anordnungselektrode (M.1, P.1, ...) an die genannte Leiterplatte (28) über die genannten leitenden Nanoleitungen (23) in einer Gruppe von Nanolöchern (21) anschließt.

2. Vorrichtung gemäß Anspruch 1, wobei eine Gruppe von leitenden Nanoleitungen (23) mehr als 10'000 und am stärksten bevorzugt mehr als 50'000 leitende Nanoleitungen umfasst und wobei die durchgehenden Löcher (21) einer Gruppe von Nanolöchern einen durchschnittlichen Durchmesser im Bereich von 10 nm bis 5 µm, bevorzugt 15-700 nm, weiter bevorzugt 20-300 nm, am meisten bevorzugt 30-100 nm aufweisen.

3. Vorrichtung gemäß irgendeinem der voranstehenden Ansprüche, wobei die genannte Membran ein Material umfasst oder im Wesentlichen daraus gebildet ist, ausgewählt aus Polycarbonat, Polyimid, Quarz, Mica, Glas, Halbleitern und Aluminiumoxid.

4. Vorrichtung gemäß irgendeinem der voranstehenden Ansprüche, wobei eine oder mehrere Anordnungselektrode(n) (M.1, P.1, ...) der genannten MEA (10) eine Vielzahl von freistehenden Nanodrähten oder Stoppeln (23a, 29) umfasst/umfassen, wobei die genannte Vielzahl von freistehenden Nanodrähten eine einzige Anordnungselektrode (M.1, oder P.1, ..) bildet.

5. Vorrichtung gemäß Anspruch 4, wobei die genannten freistehenden Nanodrähte oder Stoppeln (23a, 29) aus der oberen Oberfläche 25 der Membran (20, 20b) herausragen.

6. Vorrichtung gemäß Anspruch 4 oder Anspruch 5, wobei die genannten freistehenden Nanodrähte oder Stoppeln (23a, 29) Erweiterungen der genannten leitenden Nanoleitungen (23) sind oder umfassen.

7. Vorrichtung gemäß irgendeinem der voranstehenden Ansprüche, die weiterhin einen Controller (100) umfasst, der zum Kommunizieren mit einem Computer (200) über eine drahtlose Datenübertragung oder über ein Kabel konfiguriert ist, wobei die genannte Vorrichtung (1) weiterhin einen oder mehrere Schalter oder Wählschalter (160) umfasst, der/die durch den genannten Controller (100) zum Auswählen von Elektroden der genannten MEA zwecks Durchführung von Messungen von elektrischen Merkmalen einer Probe in der genannten Vertiefung aktiviert sein kann/können, wobei die genannte Vorrichtung weiterhin eine Mess-Teilanordnung (140) zum Messen der genannten elektrischen Merkmale umfasst.

8. Vorrichtung gemäß irgendeinem der voranstehenden Ansprüche, die eine tragbare oder mobile Vorrichtung ist und/oder die von einem Computer (200) getrennt ist, der Software zum Bedienen der Vorrichtung umfasst.

9. Vorrichtung gemäß Anspruch 7, wobei der genannte Controller (100) eine vorübergehende Speicherung (110) umfasst und zum Komprimieren von Daten geeignet ist, die von der genannten Mess-Teilanordnung (140) empfangen werden, so dass komprimierte Daten auf den genannten Computer (200) über die genannte drahtlose Datenübertragung oder über das genannte Kabel weitergeleitet werden.

10. Vorrichtung gemäß irgendeinem der Ansprüche 7-9, umfassend eine wiederaufladbare Batterie (150) und ein drahtloses Kommunikationsmodul, wobei der genannte Controller (100) zum Kommunizieren mit dem genannten Computer (200) über eine drahtlose Datenübertragung derart konfiguriert ist, dass die genannte Vorrichtung unabhängig/ohne physischen Anschluss an den genannten Computer (200) versetzt werden kann.

11. Vorrichtung gemäß irgendeinem der Ansprüche 7 bis 10, die eine Vielzahl von MEAs (10) und/oder eine Vielzahl von Vertiefungen (16) umfasst, wobei der genannte eine oder die genannten mehrere Schalter (160) einen MEA-Auswahlschalter (161) umfasst/umfassen, der eine spezifische MEA (10) der genannten Vielzahl von MEAs zum Durchführen einer Messung in der Vertiefung (16) der genannten spezifischen MEA auswählt.

12. Vorrichtung gemäß irgendeinem der voranstehenden Ansprüche, umfassend eine oder mehrere abnehmbare Einweg-Einheit(en) (16, 10) und Mehrweg-Bauteile (100, 120), wobei die genannte Vertiefung (16), die die genannte MEA (10) umfasst, Teil einer Einweg-Einheit ist, und wobei die genannte Einweg-Einheit abnehmbar auf der genannten Vorrichtung (1) derart montiert ist, dass sie nach der Einweg-Nutzung von der genannten Vorrichtung abgenommen werden kann.

13. Vorrichtung gemäß irgendeinem der Ansprüche 7-12, wobei der genannte eine oder die genannten mehreren Schalter (160) für jede MEA wenigstens zwei getrennte Anordnungselektroden-Wählschalter (165, 166), einen ersten Anordnungselektroden-Wählschalter (165) und einen zweiten Anordnungselektroden-Wählschalter (166) umfasst/umfassen, wobei die genannte Vielzahl von Anordnungselektroden eine erste Gruppe von n Anordnungselektroden (M.1, M.2, .., M.n) und eine zweite Gruppe von m Anordnungselektroden (P.1, P.2, ..., P.m) umfasst, wobei jede Elektrode der genannten ersten Gruppe von Elektroden eine getrennte und/oder eigene Schaltungsleitung zu dem genannten ersten Wählschalter (165) umfasst, und jede Elektrode der genannten zweiten Gruppe von Elektroden eine getrennte und/oder eigene Schaltungsleitung zu dem genannten zweiten Auswahlschalter (166) umfasst, und wobei der genannte Controller (100) zum Durchführen einer Messung zwischen irgendeiner ausgewählten Elektrode (M.x.) der genannten ersten Gruppe und irgendeiner ausgewählten Elektrode (P.y) der genannten zweiten Gruppe konfiguriert ist.

14. Vorrichtung gemäß Anspruch 1, wobei die genannte MEA durch ein Verfahren produziert wird, umfassend die Schritte:
(1) Bereitstellen einer Vorläufermembran (20') auf einer Leiterplatte (28), wobei die Leiterplatte (28) leitende Leitungen (27) umfasst;
(2) Herstellen von Nanoporen in geeigneten Positionen auf der Vorläufermembran (20'), während die Vorläufermembran (20') an der Leiterplatte (28) befestigt ist, wodurch eine Membran (20) erhalten wird, die Nanoporen (21) enthält;
(3) Herstellen von Nanoleitungen innerhalb der genannten Nanoporen vom Boden der Nanoporen zur oberen Oberfläche (25) der Membran; und
(4) Bilden von Anordnungselektroden der genannten MEA auf der oberen Oberfläche der genannten MEA, wodurch eine MEA erhalten wird, wobei eine einzelne Anordnungselektrode der genannten MEA durch eine Gruppe gebildet wird, die mehr als 1'000 Nanoleitungen umfasst.

## Revendications

1. Dispositif (1) comprenant un ou plusieurs puits (16) adaptés pour recevoir un échantillon avec des cellules, dans lequel chaque puits (16) comprend un réseau multi-électrodes (MEA) (10), ledit MEA (10) comprenant une membrane (20) sur laquelle une pluralité d'électrodes de réseau (M.1, P.1, ...) sont disposées pour former le réseau dudit MEA (10), dans lequel ladite membrane (20) comprend une surface supérieure (25) sur laquelle lesdites électrodes de réseau (M.1, P.1, ...) apparaissent, et une surface inférieure (26) où ladite membrane (20) est en contact avec un circuit électronique (27) connectant les électrodes de réseau (M.1, P.1, ...) à un équipement électronique aidant au fonctionnement du MEA, ***caractérisé en ce que*** la membrane (20) comprend des groupes de nanotrous traversants (21) contenant des nanolignes conductrices (23), et dans lequel un groupe de nanolignes conductrices (23) comprend plus de 1000 nanolignes et connecte électriquement une seule électrode de réseau (M.1 ou P.1, ...) dudit MEA audit circuit sur la surface inférieure (26) de ladite membrane (20), et **en ce que** ledit circuit électronique (27) est prévu sur une carte de support de circuit électronique (28), dans lequel ladite membrane (20) est fixée sur ladite carte (28) de manière à connecter électriquement une électrode de réseau (M. 1, P.1, ...) à ladite carte de circuit (28) par lesdites nanolignes conductrices (23) dans un groupe de nanotrous (21).

2. Dispositif selon la revendication 1, dans lequel un groupe de nanolignes conductrices (23) comprend plus de 10'000, et, plus préférablement, plus de 50'000 nanolignes conductrices, et dans lequel les trous traversants (21) d'un groupe de nanotrous ont un diamètre moyen dans la plage de 10 nm à 5 µm, préférablement, de 15 à 700 nm, plus préférablement, de 20 à 300 nm, le plus préférablement, de 30 à 100 nm.

3. Dispositif selon l'une quelconque des revendications précédentes, dans lequel ladite membrane comprend ou consiste essentiellement en un matériau choisi parmi le polycarbonate, le polyimide, le quartz, le mica, le verre, les semi-conducteurs et l'alumine.

4. Dispositif selon l'une quelconque des revendications précédentes, dans lequel une ou plusieurs électrodes de réseau (M.1, P.1, ...) dudit MEA (10) comprend une pluralité de nanofils ou de tiges libres (23a, 29), ladite pluralité de nanofils libres formant une seule électrode de réseau (M.1 ou P.1, ...).

5. Dispositif selon la revendication 4, dans lequel lesdits nanofils ou tiges libres (23a, 29) émergent de la surface supérieure 25 de la membrane (20, 20b).

6. Dispositif selon la revendication 4 ou revendication 5, dans lequel lesdits nanofils ou tiges libres (23a, 29) sont ou comprennent des extensions desdites nanolignes conductrices (23).

7. Dispositif selon l'une quelconque des revendications précédentes, qui comprend en outre un contrôleur (100), configuré pour communiquer avec un ordinateur (200) par une transmission de données sans fil ou par un câble, dans lequel ledit dispositif (1) comprend en outre un ou plusieurs commutateurs ou sélecteurs (160) qui peuvent être activés par ledit contrôleur (100) pour sélectionner des électrodes dudit MEA dans le but de réaliser des mesures de caractéristiques électriques d'un échantillon dans ledit puits, dans lequel ledit dispositif comprend en outre un sous-ensemble de mesure (140) pour mesurer lesdites caractéristiques électriques.

8. Dispositif selon l'une quelconque des revendications précédentes, qui est un dispositif tenu à la main ou pouvant être tenu à la main et / ou qui est séparé d'un ordinateur (200) comprenant un logiciel pour faire fonctionner le dispositif.

9. Dispositif selon la revendication 7, dans lequel ledit contrôleur (100) comprend une mémoire temporaire (110) et est adapté pour compresser les données reçues dudit sous-ensemble de mesure (140), de manière à envoyer des données compressées audit ordinateur (200) par ladite transmission de données sans fil ou par ledit câble.

10. Dispositif selon l'une quelconque des revendications 7 à 9, comprenant une batterie rechargeable (150) et un module de communication sans fil, dans lequel ledit contrôleur (100) est configuré pour communiquer avec ledit ordinateur (200) par une transmission de données sans fil, de sorte que ledit dispositif peut être déplacé indépendamment / sans connexion physique vers ledit ordinateur (200).

11. Dispositif selon l'une quelconque des revendications 7 à 10, qui comprend une pluralité de MEA (10) et / ou une pluralité de puits (16), dans lequel lesdits un ou plusieurs commutateurs (160) comprennent un commutateur de sélection de MEA (161), qui sélectionne un MEA spécifique (10) de ladite pluralité de MEA pour effectuer une mesure dans le puits (16) dudit MEA spécifique.

12. Dispositif selon l'une quelconque des revendications précédentes, comprenant une ou plusieurs entités à usage unique amovibles (16, 10) et des composants à usage multiple (100, 120), dans lequel ledit puits (16) comprenant ledit MEA (10) fait partie de l'entité à usage unique, et dans lequel ladite entité à usage unique est montée de manière amovible sur ledit dispositif (1) de façon à être retirée dudit dispositif après un usage unique.

13. Dispositif selon l'une quelconque des revendications 7 à 12, dans lequel lesdits un ou plusieurs commutateurs (160) comprennent, pour chaque MEA, au moins deux sélecteurs d'électrodes de réseau séparés (165, 166), un premier sélecteur d'électrodes de réseau (165) et un second sélecteur d'électrodes de réseau (166), dans lequel ladite pluralité d'électrodes de réseau comprend un premier groupe de n électrodes de réseau (M. 1, M.2, ..., M.n) et un second groupe de m électrodes de réseau (P.1, P.2, ..., P.m), dans lequel chaque électrode dudit premier groupe d'électrodes comprend une ligne de circuit séparée et / ou propre vers ledit premier sélecteur (165), et chaque électrode dudit second groupe d'électrodes comprend une ligne de circuit séparée et / ou propre vers ledit second sélecteur (166), et dans lequel ledit contrôleur (100) est configuré pour effectuer une mesure entre une quelconque électrode sélectionnée (M.x) dudit premier groupe et une quelconque électrode sélectionnée (P.y) dudit second groupe.

14. Dispositif selon la revendication 1, dans lequel ledit MEA est produit par un procédé comprenant les étapes suivantes :
(1) fournir une membrane précurseur (20') sur une carte de circuit imprimé (28), la carte de circuit imprimé (28) contenant des lignes conductrices (27) ;
(2) produire des nanopores sur des positions appropriées dans la membrane précurseur (20') tandis que la membrane précurseur (20') est fixée à la carte de circuit imprimé (28), obtenant ainsi une membrane (20) contenant des nanopores (21) ;
(3) produire des nanolignes à l'intérieur desdits nanopores depuis le fond des nanopores jusqu'à la surface supérieure (25) de la membrane ; et,
(4) former des électrodes de réseau dudit MEA sur la surface supérieure dudit MEA, obtenant ainsi un MEA, dans lequel une électrode de réseau individuelle dudit MEA est formée par un groupe comprenant plus de 1'000 nanolignes.
